# EUROPEAN PATENT SPECIFICATION

(11) **EP 3 679 365 B1**
(45) Date of publication and mention of the grant of the patent: **17.01.2024**
(21) Application number: 18789255.9
(22) Date of filing: 07.09.2018
(51) Int. Cl.: G01N 27/447, G01N 33/50, G01N 33/68

(54) **METHOD FOR THE ISOLATION OF BIOGENIC AMINES FROM BIOLOGICAL MATRICES**
NEUES VERFAHREN ZUR ISOLIERUNG BIOGENER AMINE AUS BIOLOGISCHEN MATRICES
NOUVEAU PROCÉDÉ D'ISOLEMENT D'AMINES BIOGÈNES À PARTIR DE MATRICES BIOLOGIQUES

(30) Priority: 08.09.2017 PL 42278817
(43) Date of publication of application: 15.07.2020
(73) Proprietor: Gdanski Uniwersytet Medyczny, 80-210 Gdansk (PL)
(72) Inventor: OLEDZKA, Ilona, PL-80-298 Gdansk (PL); MIEKUS - PURWIN, Natalia, PL-81-553 Gdynia (PL); BACZEK, Tomasz, PL-80-174 Gdansk (PL); PLENIS, Alina, PL-80-177 Gdansk (PL); KOWALSKI, Piotr, PL-80-290 (PL); GDANSKI UNIWERSYTET MEDYCZNY, PL-80-290 (PL)
(74) Representative: JWP Patent & Trademark Attorneys
(86) International application number: PCT/PL2018/000087
(87) International publication number: WO 2019/050420

(56) References cited:
- ILONA OLEDZKA ET AL: "Evaluation of various approaches to the isolation of steroid hormones from urine samples prior to FASS-MEKC analysis : CE and CEC", ELECTROPHORESIS, vol. 38, no. 12, 7 April 2017 (2017-04-07) , pages 1632-1643, XP055532199, ISSN: 0173-0835, DOI: 10.1002/elps.201600509
- TOMASZ BACZEK ET AL: "Biomedical Evaluation of Cortisol, Cortisone, and Corticosterone along with Testosterone and Epitestosterone Applying Micellar Electrokinetic Chromatography", THE SCIENTIFIC WORLD JOURNAL, vol. 2012, 1 January 2012 (2012-01-01), pages 1-8, XP055532180, DOI: 10.1100/2012/268120
- ILONA OLEDZKA ET AL: "Optimization of a Pre-MEKC Separation SPE Procedure for Steroid Molecules in Human Urine Samples", MOLECULES, vol. 18, no. 11, 13 November 2013 (2013-11-13), pages 14013-14032, XP055532223, DOI: 10.3390/molecules181114013
- ISABEL RODRIGUEZ ET AL: "Ion-pair solid-phase extraction of biogenic amines before micellar electrokinetic chromatography with laser-induced fluorescence detection of their fluorescein thiocarbamyl derivative", ELECTROPHORESIS, vol. 20, 28 July 1999 (1999-07-28), pages 1862-1868, XP055532242, DOI: 10.1002/(SICI)1522-2683(19990701)20%3A9<18 62%3A%3AAID-ELPS1862>3.0.CO%3B2-8
- JIE HUANG ET AL: "Environmentally friendly solid-phase microextraction coupled with gas chromatography and mass spectrometry for the determination of biogenic amines in fish samples", JOURNAL OF SEPARATION SCIENCE., vol. 39, no. 22, 1 November 2016 (2016-11-01), pages 4384-4390, XP055532251, DE ISSN: 1615-9306, DOI: 10.1002/jssc.201600893
- DAVID OPPOLZER ET AL: "Analytical approach to determine biogenic amines in urine using microextraction in packed syringe and liquid chromatography coupled to electrochemical detection : Determination of biogenic amines by MEPS", BIOMEDICAL CHROMATOGRAPHY., vol. 27, no. 5, 5 November 2012 (2012-11-05), pages 608-614, XP055532264, GB ISSN: 0269-3879, DOI: 10.1002/bmc.2835
- MIEKUS NATALIA ET AL: "Determination of urinary biogenic amines' biomarker profile in neuroblastoma and pheochromocytoma patients by MEKC method with preceding dispersive liquid-liquid microextraction", JOURNAL OF CHROMATOGRAPHY B: BIOMEDICAL SCIENCES & APPLICATIONS, ELSEVIER, AMSTERDAM, NL, vol. 1036, 11 October 2016 (2016-10-11), pages 114-123, XP029777636, ISSN: 1570-0232, DOI: 10.1016/J.JCHROMB.2016.10.007 cited in the application
- MIEKUS NATALIA ET AL: "Ionic liquids as signal amplifiers for the simultaneous extraction of several neurotransmitters determined by micellar electrokinetic chromatography", TALANTA, vol. 186, 17 April 2018 (2018-04-17), pages 119-123, XP085399561, ISSN: 0039-9140, DOI: 10.1016/J.TALANTA.2018.04.041
- MIEKUS NATALIA ET AL: "Cyclodextrin-modified MEKC method for quantification of selected acidic metabolites of catecholamines in the presence of various biogenic amines. Application to diagnosis of neuroblastoma", JOURNAL OF CHROMATOGRAPHY B: BIOMEDICAL SCIENCES & APPLICATIONS, ELSEVIER, AMSTERDAM, NL, vol. 1003, 10 September 2015 (2015-09-10), pages 27-34, XP029284283, ISSN: 1570-0232, DOI: 10.1016/J.JCHROMB.2015.09.003 cited in the application

## Description

The subject of invention is a novel method for the isolation of biogenic amines from urine, which applies ionic liquids for solid-phase microextraction (SPME) of biogenic amines prior to their separation with micellar electrokinetic chromatography (MEKC).

The determination of biogenic amines in biological material has evoked significant interest due to the large diagnostic importance of those compounds. Since each biological material is a complex matrix with heterogeneous chemical composition, the analytes must be isolated selectively and carefully. This particular stage of the experiment tends to be the most problematic in the opinion of the scientists. The most frequently studied matrix for the quantitative analysis of the biogenic amines is urine and - less often - plasma or blood serum samples. Various extraction methods can be employed for the selective isolation of biogenic amines, such as liquid-liquid extraction (LLE), dispersive liquid-liquid microextraction (DLLME), solid-phase extraction (SPE) which applies various types of SPE coatings such as C18, HLB, MCX, SCX. In some cases, extraction techniques are preceded by chemical or enzymatic hydrolysis which causes the release of the tested compounds from the fusion with plasma proteins in case of the analysis of serum samples, or from its conjugate (glucoronate or sulfonate) in case of the analysis of urine samples. One must however note that this stage is always time-consuming and often brings no anticipated growth in method detectability.

With regards to the separation techniques applied during the analysis of biogenic amines, attention must be paid to the fact that the most sensitive method is the combination of mass spectrometry (MS) detection and a separation technique, which enables determination of biogenic amines at the level of several ng, or even pg. However, the use of MS detection is relatively costly, and for this reason, it is not generally available in analytical labs. Another sensitive detector applied for the determination of biogenic amines is laser-induced fluorescence (LlF) detector. It is characterized by sensitivity approximately the same as MS detector, but it requires usually the application of additional stage of the experiment - based on the derivatization of analytes prior to their determination. Agents used for the derivatization are cost-intensive, can be toxic for human and rather not environmentfriendly. Thus, the majority of methods concerning the determination of biogenic amines are based on UV detectors which, on the one hand are the least sensitive while on the other hand being relatively cheap and popular. For this reason, the methods based on UV detection are attractive to be used in routine laboratory analyses.

Therefore, the analysts presently focus on searching for approaches which would improve the sensitivity of the method already at the stage of the extraction of analytes from the matrix or at the stage of the on-line signal enhancement during the separation of biogenic amines.

It must also be pointed out that due to the diverse properties of individual biogenic amine, already conducted studies that could be found in scientific literature mostly concerned with the determination of from 2 to 5 compounds at most, which are characterized by a similar physicochemical properties. The method which is the subject of the presented in here invention allows simultaneous extraction of as many as 7 biogenic amines.
In the scope of applying ionic liquids within the analysis of biogenic amines.
Biogenic amines were also determined with the use of other ionic liquids which were applied at different stages of the analytical methodologies elaboration. The application of HMIMPF6, as a compound which improves the extraction during the determination of the content of 5-hydroxyindoles (*amines different to tested amines*) in the plasma samples, enabled to obtain satisfactory improvement of analytes' signals as well as values of the limits of determination. Two other ionic liquids, which chemical structure are based on imidazole ring (C₁₆MlmCl and C₁₂MlmCl), allowed the improvement of separation of dopamine, norepinephrine, adrenaline and normetanephrine. Despite lowering LOD values (limit of detection of the method) these procedures did not enable the quantitative analysis of biogenic amines in the tested sample.

Ionic liquids excellently fit in the postulates of "green chemistry", mainly due to their low toxicity, diversity of chemical structures and physicochemical properties. They may fulfil the role of universal agents applicable in many areas of science, including toxicology, organic chemistry and analytical chemistry.

Ionic liquids have also been applied in the form of a composite, immobilized on a graphene-chitosan plate, in the method of biosensoric measurement of amine concentration, including dopamine, obtaining LOD at concentration 0,04 µM/L.

Ionic liquids are becoming an object of interest, especially in the case of bioanalytical methods which deal with the small sample volumes and low concentrations of studied compounds. This type of methods includes electromigration techniques which are cheap in exploitation and environment friendly, but they require application of effective methods of analyte extraction from biological samples.

Nowadays, with continuously increasing analytical demands it is necessary to:
- reduce the usage of biological samples: having only a small volume of biological sample it is necessary to obtain a number of clinically significant information about compounds, identified as biomarkers, and presented in trace amounts in the samples
- reduce or eliminate the toxic reagents: due to the pollution of the environment and danger for an analyst performing the experiments
- simplify experimental procedures in order to avoid additional costs, minimize the time necessary to carry out the analysis and provide reliable results of tests in a relatively short time.

The subject of researches was to improve the efficiency of the method of solid phase microextraction (SPME) through the application of ionic liquids. SPME was chosen due to the fact that it is a high-throughput technique (possibility to simultaneously prepare up to 96 samples), featured by low usage both of reagents (organic solvents), and biological matrix as well as high automation. However, poor range of commercially available SPME coatings that could be applied for the extraction of many biologically significant compounds (from the group of biogenic amines (adrenaline, noradrenaline, dopamine and serotonin) and their precursor amino acids (L-tyrosine, L-tryptophan, L-DOPA)) at the same time, led us to propose very selective additives to desorbent phase - ionic liquids. Analyses conducted by us allowed to optimize the method based on SPME during a simultaneous extraction of seven compounds from biological samples with the application of ionic liquid-enriched desorbent. All the tested ionic liquids belonged to imidazole derivatives, differing by an anion which formed part of a given liquid (1-butyl-3-methylimidazolium tetrafluoroborate, 1-ethyl-3-methylimidazolium tetrafluoroborate and 1-ethyl-3-methylimidazolium bis(trifluoromethylsulfonyl)imide).

The objective of the presented invention is to provide a new method for the extraction of analytes with SPME and ionic liquids as an additive in the desorbent phase, which may be applied for the extraction procure for seven analytes: biogenic amines and their precursor amino acids from biological matrices. Unexpectedly, the problem of low efficiency of analyte extraction from biological material has been solved by the hereby invention.

The subject of the invention is a new method for the isolation of biogenic amines from biological material, where extraction of biogenic amines and their precursor amino acids: L-tryptophan, serotonin, dopamine, L-tyrosine, L-DOPA, noradrenaline, adrenaline from urine occurs through:
- activation of SPME coating (PS-DVB) with a mixture composed of methanol: water (50:50; v/v) - 30 minutes; then short rinsing (10 seconds) with deionized water occurs;
- extraction of acidified urine samples with pH 3, containing the tested analytes, extraction time -90 minutes;
- washing the SPME coatings with deionized water - 10 seconds;
- desorption of analytes for 90 minutes with 2 mL 1-ethyl-3-methylimidazolium tetrafluoroborate -enriched methanol in the concentration of 20 ng/mL;
- evaporation of desorbent phase in a device for evaporation of samples under a decreased pressure, at temperature 40°C;
- dissolving of the dry residue in 50 µL of sample buffer 2 mM sodium tetraborate;
- electrophoretic analysis (by the MEKC).

Methods of isolation, CE separation buffer consisted of 10 mM sodium tetraborate decahydrate, 30 mM SDS, 15% *(v*/*v)* of methanol and 25 mM of α-cyclodextrin.

Isolation method, where the mixture was brought to pH 9,36 with the use of 1 M NaOH.

### Figures description:

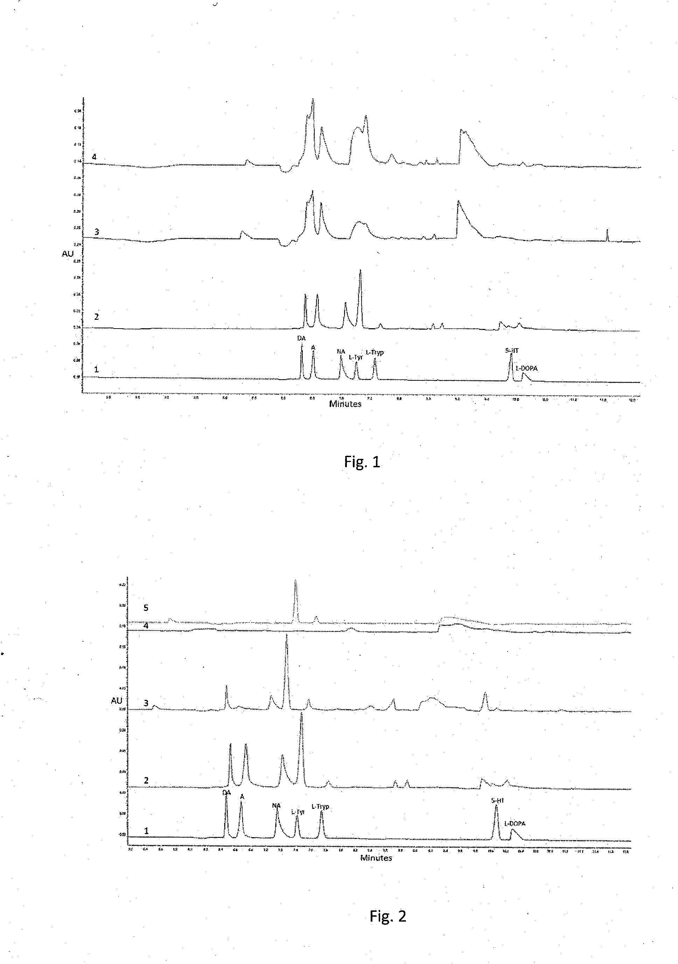

**Fig. 1** - presents *the electropherogram of reference sample containing biogenic amines at the concentration of 20 µg*/*mL (1) and electropherograms of the samples after the SPME extraction (each analyte at the concentration of 20 µg*/*mL) with the use of various desorbents: (2) Methanol (3) Acetonitrile, (4) Acetone*
   *Legend: DA* - *dopamine; A* - *adrenaline; NA* - *noradrenaline; L-Tyr* - *L-tyrosine, L-Tryp* - *L-tryptophan; 5-HT-serotonin; L-DOPA* - *L-DOPA*
**Fig. 2** - presents *the electropherogram of reference sample containing biogenic amines at the concentration of 20 µg*/*mL (1) and electropherograms of the samples after the SPME extraction (each compound at the concentration of 20 µg*/*mL) with the use of methanol as desorbent in different conditions of pH (2-5): (2) pure methanol, (3) methanol* + *0.1 M HNO₃ (pH 2), (4) methanol+ 2.5* % *NH₄OH (pH 8), (5) methanol* + *0.1 N NaOH (pH 9.5).*
   *Legend: DA* - *dopamine; A* - *adrenaline; NA* - *noradrenaline; L-Tyr* - *L-tyrosine, L-Tryp* - *L-tryptophan; 5-HT-serotonin; L-DOPA* - *L-DOPA*

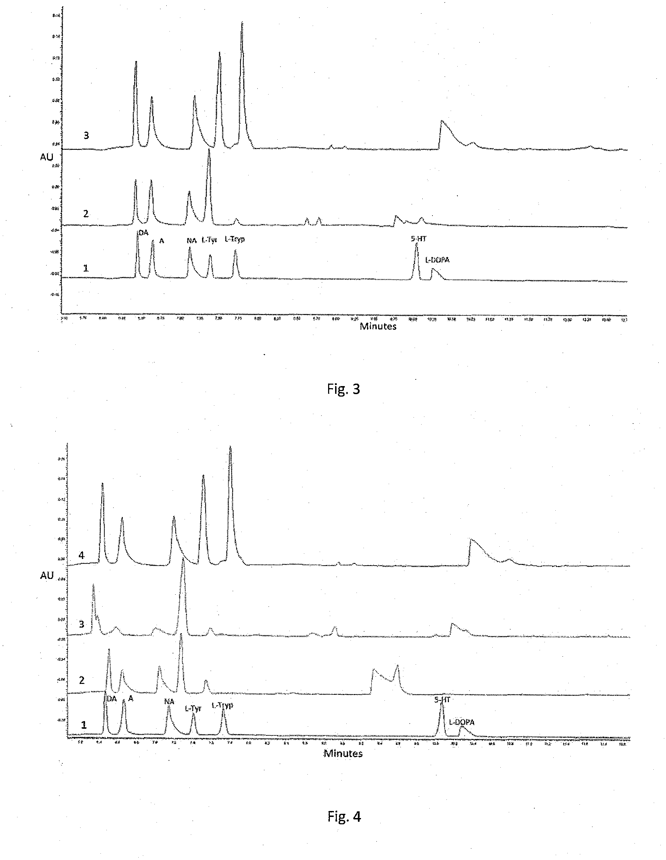

**Fig. 3** - presents *the electropherogram of reference sample containing biogenic amines at the concentration of 20 µg*/*mL (1) and electropherograms of the samples after the SPME extraction (each analyte at the concentration of 20 µg*/*mL) with the use of various SPME coatings: C18 (2) and PS-DVB (3).*
   *Legend: DA* - *dopamine; A* - *adrenaline; NA* - *noradrenaline; L-Tyr* - *L-tyrosine, L-Tryp* - *L-tryptophan; 5-HT-serotonin; L-DOPA* - *L-DOPA*
**Fig. 4** - presents *the electropherogram of reference sample containing biogenic amines at the concentration of 20 µg*/*mL (1) and electropherograms of the samples after the SPME extraction (each analyte at the concentration of 20 µg*/*mL) with the use of methanol as desorbent phase (2-4): (2) 40 min adsorption*/*desorption, (3) 60 min adsorption*/*desorption (4) 90 min adsorption*/*desorption*
   *Legend: DA* - *dopamine; A* - *adrenaline; NA* - *noradrenaline; L-Tyr* - *L-tyrosine, L-Tryp* - *L-tryptophan; 5-HT-serotonin; L-DOPA* - *L-DOPA*

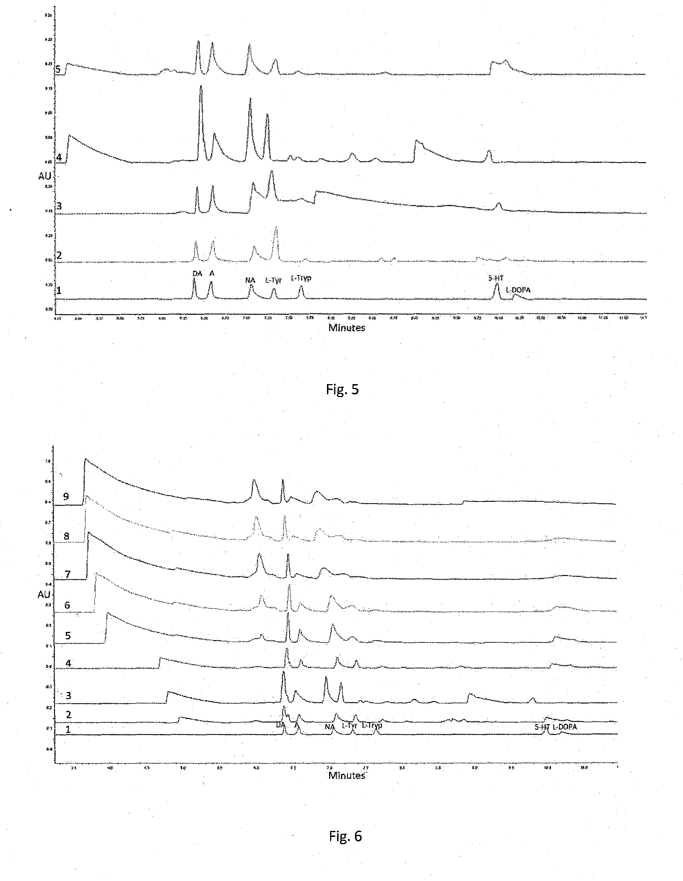

**Fig. 5** - presents *the electropherogram of reference sample containing biogenic amines at the concentration of 20 µg*/*mL (1) and electropherograms of the samples after the SPME extraction (each analyte with concentration of 20 µg*/*mL) with the use of various ionic liquids in a desorbent phase (2-5): (2) Methanol, (3) Methanol* + *IL1 (20 ng*/*mL), (4) Methanol* + *IL2 (20 ng*/*mL), (5) Methanol* + *IL3 (20 ng*/*mL)*
   *Legend: IL1* - *1-butyl-3-methylimidazolium tetrafluoroborate, IL2* - *1-ethyl-3-methylimidazolium tetrafluoroborate, IL3* - *1-ethyl-3-methylimidazolium bis(trifluoromethylsulfonyl)imide*
   *DA* - *dopamine; A* - *adrenaline; NA* - *noradrenaline; L-Tyr* - *L-tyrosine, L-Tryp* - *L-tryptophan; 5-HT* - *serotonin; L-DOPA* - *L-DOPA*
**Fig 6** - presents *the electropherogram of reference sample containing biogenic amines at the concentration of 20 µg*/*mL (1) and electropherograms of the samples after the SPME extraction (each analyte in the concentration of 20 µg*/*mL) with the use of various concentration of ionic liquid IL2 (1-ethyl-3-methylimidazolium tetrafluoroborate) in the methanol (2-9): (2) Methanol* + *IL2 (10 ng*/*mL), (3) Methanol + IL2 (20 ng*/*mL), (4) Methanol+ IL2 (50 ng*/*mL), (5) Methanol* + *IL2 (100 ng*/*mL), (6) Methanol+ IL2 (200 ng*/*mL), (7) Methanol + IL2 (300 ng*/*mL), (8) Methanol+ IL2 (400 ng*/*mL), (9) Methanol* + *IL2 (500 ng*/*mL). Legend: DA* - *dopamine; A* - *adrenaline; NA* - *noradrenaline; L-Tyr* - *L-tyrosine, L-Tryp - L-Tryptophan; 5-HT-serotonin; L-DOPA* - *L-DOPA*

The invention presents an example of performance , not constituting its limitation.

### 1. Data concerning patent description

### 1.1.Completion of experiment

### 1.1.1. The preparation of reference solutions and reagents for the analysis

Dopamine (DA), adrenaline (A), noradrenaline (NA), L-tryptophan (L-Tryp), L-tyrosine (L-Tyr), serotonin (5-HT) and L-DOPA, as well as ionic liquids: 1-butyl-3-methylimidazolium tetrafluoroborate, 1-ethyl-3-methylimidazolium tetrafluoroborate and 1-ethyl-3-methylimidazolium bis(trifluoromethylsulfonyl)imide. Methanol, sodium dodecyl sulphate (SDS), α-cyclodextrin (α-CD) and sodium tetraborate decahydrate (borax), regenerating liquid for electrophoresis device, deionised water. All agents with analytical purity were applied without further purification. Highly pure water was obtained from Milli-Q. equipment

### 1.1.1.1. Preparation of reference solutions of biogenic amines

Reference biogenic amines solutions were prepared in the concentration of 1 mg/mL in the following manner. Exactly 1.0 mg of each reference substance was weighed on the analytical balance in separate Eppendorf tubes with 1.5 mL capacity. Subsequently, reference solutions of the substances: L-tryptophan, serotonin and dopamine were prepared through dissolving each substance separately in 1.0 mL of methanol; while solutions of L-tyrosine, L-DOPA, noradrenaline and adrenaline were prepared through dissolving each substance separately in 950 µL of methanol with the addition of 50 µL of the 0.1 M HCl. The addition of 50 µL of the 0.1 M hydrochloric acid into the reference solutions of four indicated biogenic amines improved their solubility in methanol. All solutions were mixed with the use of vortex for approx. 2 minutes and then subjected to the ultrasounds in the ultrasonic bath for 5-15 minutes. The above basic reference solutions were stored in a freezer at the temperature of 20 °C, protecting them from light for two weeks. Working solutions of all analytes in the concentrations of 20 and 0.25 µg/mL were prepared daily by adequate dilution of stock solution of a given analyte in water. Working solutions were stored at 4°C in closed container up to 8-10 hours.

### 1.1.1.2. The elaboration of the activation solution for SPME

SPME coating activation solution was prepared (methanol/water 50:50, *v*/*v).* For this purpose, 15 mL of deionised water and 15 mL of methanol was mixed in the glass volumetric flask with 50 mL capacity and manually shaken for two minutes. The activation solution for SPME was stored at the room temperature in the fume cupboard.

### 1.1.1.3. Preparation of desorption solutions that contained ionic liquids.

The desorption solution containing ionic liquid (1-ethyl-3-methylimidazolium tetrafluoroborate) in the concentration of 20 ng/mL in methanol was elaborated through measuring 1.0 µL of ionic liquid ([EMIM]⁺[BF₄]⁻ m.cz. 197,97) and dissolving it in 10 mL of methanol.

### 1.1.1.4. Preparation of a buffer for dissolving samples after extraction of analytes from the urine samples

2 mM of sodium tetraborate was prepared as a buffer solution for dissolving samples after analyte' extraction from the urine samples and after desorbent evaporation. For this purpose, 1 mL of 100 mM sodium tetraborate decahydrate solution was placed in the volumetric flask with 50 mL capacity and filled up with deionised water up to the mark.

### 1.1.1.5. Preparation of the separation buffer composition for the electrophoretic analysis

Separation buffer (BGE) composed of borax (10 mM), SDS (30 mM), α-cyclodextrin (25 mM) and methanol (15 %, *v*/*v)* was applied during the analytes separation with the micellar electrokinetic chromatography method (MEKC). It was elaborated during several stages. In the first stage exactly 1,216 g of α-cyclodextrin was placed in the volumetric flask with capacity of 50 mL, then 5 mL of borax solution in the concentration of 100 mM, 7.5 mL of methanol and 30 mL of deionised water were added. Subsequently, the volumetric flask was placed for 10 minutes in an ultrasonic bath. In the further stage, 200 mM of SDS solution was added to the solution located in the volumetric flask. Finally, the solution of separation buffer was brought to pH 9.36 with the use of 1 M NaOH.

### 1.1.2. The urine samples preparation procedure

### 1.1.2.1. Samples collection

Urine samples were acidified to pH 3 with the use of 6 M HCl in the following manner: 50 µL of 6 M HCl was added to each test tube with capacity of 15 mL and the urine sample (10 mL) was added. The solution was stored in the temperature -80 °C until the time of urine sample analysis (it concerns real samples, used in the method being the subject of invention).

During the elaboration of the biogenic amines' extraction procedure, the urine samples were enriched with each analyte at the concentration of 20 µg/mL. Subsequently, the urine sample was acidified to pH 3 with the use of 6 M HCl (this concerns the validation and the optimization of a method being the subject of invention).

### 1.1.2.2. SPME extraction

The 96-blade SPME, in which PS-DVB was applied as a stationary phase, was carried out. SPME procedure consisted of several stages: conditioning of the SPME coatings with the mixture of methanol : water (50:50; *v*/*v),* adsorption of the analytes from the sample and elution of the analytes (desorption). Short, 10-second rinsing with deionized water was applied between each stage in order to prevent the transfer of solutions and eliminate interfering polar substances from the desorbent phase.

The procedure of extraction of analytes from biological sample with the use of the SPME method:
- Activation of PS-DVB coatings with the mixture of methanol : water (50:50; *v*/*v) -* 30 minutes; then short rinsing (10 seconds) with deionized water.
- Extraction of acidified urine samples (pH 3) containing the compounds of interest from 1 mL of urine sample - time of extraction: 90 minutes
- Washing the SPME coatings with deionized water (10 seconds).
- Desorption of analytes for 90 minutes with the 1 mL methanol solution enriched with the ionic liquid (1-ethyl-3-methylimidazolium tetrafluoroborate) at the concentration of 20 ng/mL
- Evaporation of the desorbent under a decreased pressure at the temperature 40 °C
- Dissolution of dry residue in 50 µL of the sample buffer
- Electrophoretic analysis (by MEKC)

### 1.1.3. Electrophoretic analysis

The electrophoretic apparatus equipped with the autosampler, diode array detector (DAD), temperature-controlling device as well as system for the data collection provided by the manufacturer was applied. The parameters of electrophoretic analysis with the use of the micellar electrokinetic chromatography technique (MEKC) were as follows: non-modified silica capillary (internal diameter -75 µm and total length- 60.2 cm); λ = 200 nm; injection time 8 s; applied voltage 22 kV; analysis temperature 22 (± 0.1) °C. Between each run, the conditioning of capillary was performed in order to ensure analyses repeatability, which covered rinsing with 0.1 M NaOH for 1 minute under the pressure of 50 psi (I stage), and subsequently rinsing the capillary with Milli-Q. water for 1 minute under the pressure of 50 psi (II stage) and finally with running buffer for 1 minute under the pressure 50 psi (III stage). The mixture composed of 10 mM of sodium tetraborate, 30 mM SDS, 15 % *(v*/*v)* methanol and 25 mM α-cyclodextrin was used as a back ground electrolyte (BGE). The BGE was brought to pH 9.36 with the use of 1 M of NaOH.

### The critical parameter for MEKC analysis

| **PARAMETER** | **VALUE OF PARAMETER** |
|---|---|
| Capillary internal diameter | 75 µm |
| Capillary length | 60,2 cm |
| Injection time | 8 s |
| Time of analysis | 15 minutes |
| UV wavelength | 200 nm |
| Voltage | 22 kV |
| BGE (running buffer) | 10 mM borax, 30 mM SDS, 25 mM α-CD, 15 % *(v*/*v)* methanol (pH 9.36) |

### 1.2.Result of analysis

### 1.2.1. Selection of SPME desorbent

Three different organic solvents were tested - methanol, acetonitrile and acetone, of which methanol turned out to be the most optimal desorbent for the tested biogenic amines from the urine samples. These data are confirmed by the results presented on Fig. 1., which illustrate electropherograms obtained while the analytes from the urine samples underwent SPME extraction with the use of indicated desorbents.

### 1.2.2. Selection of appropriate pH for the SPME desorbent

At the beginning of the study, pH in the range between 2 and 9.5 was tested and the obtained results have been presented on Fig. 2, which confirms that the greatest extraction efficiency of 5-HT and L-Tyr were obtained while the acidic pH was used, but in the case of other analytes, these conditions were ineffective. However, alkalization of methanol by addition of 2.5 % NH₄OH to pH 8.0 or 0.1 M NaOH to pH 9.5 worsened the extraction efficiency for biogenic amines. Thus, change of pH did not brought satisfactory improvement of the extraction efficiency for all the analytes and for this reason, pure methanol has been selected without modifying pH, for further testing.

### 1.2.3. Selection of SPME coating

Two different commercially available stationary phases (C18 and PS-DVB) for the isolation of biogenic amines from the acidified urine samples (pH 3) were taken into account. The experimental data confirmed that the PS-DVB coatings is more appropriate for studied compounds extraction (DA, A, NA, L-Tryp and L-Tyr) (Fig. 3.)

### 1.2.4. Selection of time of adsorption and desorption

The optimal duration of both: the adsorption and desorption times for the biogenic amines, with the use of PS-DVB SPME, from urine samples were estimated through the extraction of analytes from the urine samples, enriched with the tested analytes (each with concentration of 20 µg/mL), which were extracted in acidic environment (pH 3) and desorbed with the pure methanol in different time from 40 to 90 minutes. The obtained experimental data confirmed that the time of adsorption/desorption determines the effectiveness of the extraction (Fig. 4), whilst the highest efficiency was obtained when both: the absorption time and the desorption times amounted 90 minutes.

### 1.2.5. Selection of the type of ionic liquid in the desorbent phase

Within the study, three of ionic liquids were tested: 1-butyl-3-methylimidazolium tetrafluoroborate (IL1), 1-ethyl-3-methylimidazolium tetrafluoroborate (IL2) and 1-ethyl-3 methylimidazolium bis(trifluoromethanesulfonyl)imide (IL3), of which each was added to the methanol in the concentration of 20 ng/mL. Figure 5 presents electropherograms of the enriched with seven biogenic amines (each at the concentration of 20 µg/mL) urine samples after PS-DVB SPME extraction for 90 minutes. The obtained data confirmed that 1-ethyl-3-methylimidazolium tetrafluoroborate (IL2) is the most effective ionic liquid to be added to methanol for biogenic amines extraction, among the tested ionic liquids.

### 1.2.6. Selection of concentration of 1-ethyl 3-methylimidazolium tetrafluoroborate in methanol as desorbent phase

The impact of concentration of ionic liquid - 1-ethyl 3-methylimidazolium tetrafluoroborate - in the methanol on the extraction efficiency of the biogenic amines from the human urine samples was assessed. For this purpose, 1-ethyl-3-methylimidazolium tetrafluoroborate was added to pure methanol at the concentration ranged from 10 ng/mL to 500 ng/mL. The obtained data indicated that the highest extraction efficiency for all studied compounds was obtained when the concentration of ionic liquid in methanol amounted 20 ng/mL. Lower concentration of ionic liquid as well as an increase of its concentration above 20 ng/mL worsens the effectiveness of the extraction (Fig. 6)

### Summary of outcomes

The conducted tests enabled to evaluate the most optimal conditions for the biogenic amines' biological sample preparation. A significant improvement of recovery with reference to all seven tested compounds (dopamine, adrenaline, noradrenaline, L-tyrosine, L-tryptophan, serotonin and L-DOPA) was noted. The most optimal ionic liquid was chosen in the study was 1-ethyl-3-methylimidazolium tetrafluoroborate in the concentration of 20 ng/mL in the desorbent phase (methanol). This ionic liquid- enriched methanol was the most optimal desorbent in the procedure of isolating biogenic amines from the urine samples by means of solid phase microextraction (SPME). The addition of ionic liquid caused an increase in extraction efficiency for all analytes which, in turn, enabled the analysis of trace amounts of biogenic amines in the urine samples with the use of MEKC technique with UV/VIS detection.

The innovativeness of the experiments carried out by the originators of the hereby invention involves a pioneer application of ionic liquid as an addictive to the desorbent phase in the SPME during the simultaneous isolation of seven biogenic amines from the urine samples. Taking into consideration a large lability (photo- and thermal-sensitivity) of the selected group of analytes, as well as their diverse physicochemical properties, methods applied for their isolation from biological samples often possesses many disadvantages (they do not consider the degree of complexity of the matrix, conditions of correct storage of the samples or they do not allow the simultaneous isolation of a numerous group of analytes). The proposed SPME-based methodology with an addition of ionic liquid allows for a fast and more precise as well as more environmental friendly determination of biogenic amines in the urine samples.

### 2.1 Comparison of the methods according to the invention with the earlier elaborated methods by the originators of the hereby invention

| **Analytical method of detection** | **Biological material** | **Method of extraction of analytes from the biological matrix** | **Tested analytes** | **Obtained limit of quantification (LOQ) [µg/mL]** | **Obtained limit of detection (LCD) [µg/mL]** |
|---|---|---|---|---|---|
| Natalia Miekus, Piotr Kowalski, Ilona Ol 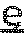 dzka, Alina Plenis, Ewa Bień, Aleksandra Mi 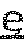 kus, Ma 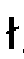 gorzata Krawczyk, Elżbieta Adamkiewicz-Drożyńska, Tomasz B 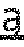 czek, Cyclodextrin-modified MEKC method for quantification of selected acidic metabolites of catecholamines in the presence of various biogenic amines : application to diagnosis of neuroblastoma, J. Chromatogr. B., 2015 (1003) 27-34 | | | | | |
| MEKC with UV detection (λ 200 nm) | Urine (1 mL) | Liquid-Liquid Extraction (LLE) with the application of 1 mL of ethyl ether | VMA | 0.5 | 0.10 |
| | | | HVA | 0.5 | 0.10 |
| | | | DOPAC | 0.5 | 0.25 |

| Natalia Mi 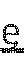 kus, Ilona Ol 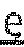 dzka, Alina Plenis, Piotr Kowalski, Ewa Bień, Aleksandra Mi 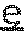 kus, Matgorzata Anna Krawczyk, Elżbieta Adamkiewicz-Drożyńska, Tomasz B 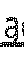 czek, Determination of urinary biogenic amines' biomarker profile in neuroblastoma and pheochromocytoma patients by MEKC method with preceding dispersive liquid-liquid micro extraction, J. Chromatogr. B 2016; vol. 1036-1037, s. 114-123 | | | | | |
|---|---|---|---|---|---|
| MEKC with UV detection (λ 200 nm) | Urine (1 mL) | Dispersive liquid-liquid microextraction | DA | 0.5 | 0.15 |
| | | | A | 0.5 | 0.15 |
| | | | NA | 0.5 | 0.15 |
| | | | L-Tryp | 0.5 | 0.15 |
| | | | L-Tyr | 0.5 | 0.15 |
| | | | 5-HT | 0.5 | 0.15 |
| | | | L-DOPA | 0.5 | 0.15 |

| Presented methodology with the use of ionic liquid | | | | | |
|---|---|---|---|---|---|
| MEKC with UV detection (λ 200 nm) | Urine (1 mL) | Solid phase microextraction (SPME) with methanol as desorbent with an addition of ionic liquid | DA | 0.25 | 0.08 |
| | | | A | 0.25 | 0.08 |
| | | | NA | 0.25 | 0.08 |
| | | | L-Tyr | 0.25 | 0.08 |
| | | | L-Tryp | 0.25 | 0.08 |
| | | | 5-HT | 0.5 | 0.15 |
| | | | L-DOPA | 0.5 | 0.15 |

## Claims

1. The method of the isolation of biogenic amines from biological material is **characterized by** the fact that the extraction of biogenic amines and their precursor amino acids: L-tryptophan, serotonin, dopamine, L-tyrosine, L-DOPA, noradrenaline, adrenaline from urine occurs through:
- activation of solid phase micro-extraction (SPME) polystyrene-divinylbenzene (PS-DVB) coatings with the mixture of methanol : water
(50:50; *v*/*v)* - 30 minutes; then short rinsing with deionized water for 10 seconds;
- extraction of acidified urine samples with pH 3, containing the tested analytes, extraction time - 90 minutes;
- washing of SPME coatings with deionized water - 10 seconds;
- desorption of analytes for 90 minutes with 2 mL 1-ethyl-3-methylimidazolium tetrafluoroborate -enriched methanol in the concentration of 20 ng/mL;
- evaporation of desorbent under a decreased pressure, at temperature of 40 °C;
- dissolution of the dry residue in 50 µL of sample buffer 2 mM sodium tetraborate;
- electrophoretic analysis by micellar electrokinetic chromatography (MEKC).

2. The method of the isolation according to claim 1 is **characterized by** the fact that in the electrophoretic analysis, the running buffer was composed of the mixture of 10 mM sodium tetraborate, 30 mM SDS, 15 % (v/v) methanol and 25 mM α-cyclodextrin.

3. The method of the isolation according to claim 6 is **characterized by** the fact the separation buffer mixture was adjusted to pH 9.36 with the use of 1 M NaOH.

## Patentansprüche

1. Verfahren zur Isolierung biogener Amine aus biologischem Material **dadurch gekennzeichnet, dass** die Extraktion von biogenen Aminen und ihren Vorläuferaminosäuren: L-Tryptophan, Serotonin, Dopamin, L-Tyrosin, L-DOPA, Noradrenalin, Adrenalin aus dem Urin durch:
- Aktivierung von Festphasenmikroextraktions-(SPME)-Polystyrol-Divinylbenzol-(PS-DVB)-Beschichtungen mit einer Mischung aus Methanol: Wasser (50:50; Vol./Vol.) - 30 Minuten; anschließend kurzes Spülen mit deionisiertem Wasser über 10 Sekunden;
- Extraktion der die untersuchten Analyten enthaltenden angesäuerten Urinproben mit pH 3, Extraktionszeit - 90 Minuten;
- Waschen der SPME-Beschichtungen mit deionisiertem Wasser - 10 Sekunden;
- Desorption der Analyten über 90 Minuten mit 2 mL 1-Ethyl-3-methylimidazoliumtetrafluoroborat-angereichertem Methanol in der Konzentration von 20 ng/mL;
- Verdampfung des Desorptionsmittels unter vermindertem Druck, bei einer Temperatur von 40°C;
- Auflösen des Trockenrückstandes in 50 µL Probenpuffer 2 mM Natriumtetraborat;
- elektrophoretische Analyse durch mizellare elektrokinetische Chromatographie (MEKC) erfolgt.

2. Verfahren zur Isolierung nach Anspruch 1 **dadurch gekennzeichnet, dass** bei der elektrophoretischen Analyse der Laufpuffer aus einer Mischung aus 10 mM Natriumtetraborat, 30 mM SDS, 15% (Vol./Vol.) Methanol und 25 mM α-Cydodextrin bestand.

3. Verfahren zur Isolierung nach Anspruch 1 **dadurch gekennzeichnet, dass** die Trennpuffermischung unter Verwendung von 1 M NaOH auf pH 9,36 eingestellt wurde.

## Revendications

1. Le procédé d'isolement d'amines biogènes à partir de matériel biologique est **caractérisé par le fait que** l'extraction d'amines biogènes et de leurs acides aminés précurseurs: le L-tryptophane, la sérotonine, la dopamine, la L-tyrosine, la L-DOPA, la noradrénaline, l'adrénaline urinaire se produit par:
- activation des revêtements de polystyrène-divinylbenzène (PS-DVB) de microextraction en phase solide (SPME) avec le mélange méthanol: eau (50:50 ; v/v) - 30 minutes; puis rinçage court à l'eau désionisée pendant 10 secondes;
- extraction d'échantillons d'urine acidifiés à pH 3, contenant les analytes testés, temps d'extraction - 90 minutes;
- lavage des revêtements SPME à l'eau désionisée - 10 secondes;
- désorption des analytes pendant 90 minutes avec 2 ml de tétrafluoroborate de 1-éthyl-3-méthylimidazolium - méthanol enrichi à la concentration de 20 ng/mL;
- évaporation du désorbant sous une pression réduite, à une température de 40 °C;
- dissolution du résidu sec dans 50 µL de tampon échantillon 2 mM de tétraborate de sodium;
- analyse électrophorétique par chromatographie électrocinétique micellaire (MEKC).

2. Le procédé d'isolement selon la revendication 1 est **caractérisé par le fait que**, dans l'analyse électrophorétique, le tampon de fonctionnement était composé du mélange de 10 mM de tétraborate de sodium, de 30 mM de SDS, de 15 % *(v*/*v)* de méthanol et de 25 mM d'a-cyclodextrine.

3. Le procédé d'isolation selon la revendication 1 est **caractérisé par le fait que** le mélange de tampon de séparation a été ajusté à un pH de 9,36 avec l'utilisation de NaOH 1 M.
